# EUROPEAN PATENT SPECIFICATION

(11) **EP 3 081 650 B1**
(45) Date of publication and mention of the grant of the patent: **06.03.2019**
(21) Application number: 14870526.2
(22) Date of filing: 10.12.2014
(51) Int. Cl.: C12R 1/01, C12R 1/44, F24F 3/16, C12Q 1/00, C12Q 1/02, C12Q 1/18, C12N 1/20, A61L 9/00

(54) **ANTIMICROBIAL AGENT SCREENING METHOD**
SCREENING-VERFAHREN EINES ANTIMIKROBIELLEN WIRKSTOFFS
PROCÉDÉ DE CRIBLAGE D'UN AGENT ANTIMICROBIEN

(30) Priority: 10.12.2013 KR 20130153141; 10.12.2013 KR 20130153142; 17.12.2013 KR 20130156930; 17.12.2013 KR 20130156929; 17.12.2013 KR 20130156928; 17.12.2013 KR 20130156927; 30.12.2013 KR 20130166781; 30.12.2013 KR 20130166782; 11.03.2014 KR 20140028278; 11.03.2014 KR 20140028279; 11.03.2014 KR 20140028280; 11.03.2014 KR 20140028277
(43) Date of publication of application: 19.10.2016
(73) Proprietor: Hyundai Motor Company, Seoul 137-938 (KR)
(72) Inventor: LEE, Tae Hee, Hwaseong-si Gyeonggi-do 445-776 (KR); KIM, Ji Wan, Yongin-si Gyeonggi-do 446-912 (KR); PARK, So Yoon, Seoul 156-777 (KR)
(74) Representative: Isarpatent
(86) International application number: PCT/KR2014/012111
(87) International publication number: WO 2015/088237

(56) References cited:
- WO-A1-96/14882
- WO-A2-02/27018
- DE-A1- 19 750 128
- JP-A- 2008 523 820
- KR-A- 20070 112 850
- ROBERT B. SIMMONS ET AL: "The Occurrence and Persistence of Mixed Biofilms in Automobile Air Conditioning Systems", CURRENT MICROBIOLOGY, vol. 39, no. 3, 1 September 1999 (1999-09-01), pages 141-145, XP055260274, Boston ISSN: 0343-8651, DOI: 10.1007/s002849900435
- NINA DIEKMANN ET AL: "Microbial communities related to volatile organic compound emission in automobile air conditioning units", APPLIED MICROBIOLOGY AND BIOTECHNOLOGY, vol. 97, no. 19, 23 October 2013 (2013-10-23), pages 8777-8793, XP055381426, DE ISSN: 0175-7598, DOI: 10.1007/s00253-012-4564-4
- MARIKO TAKEUCHI ET AL.: 'Union of the genera Microbacterium Orla-Jensen and Aureobacterium Collins et al. in a redefined genus Microbacterium' INT. J. SYST. BACTERIOL. vol. 48, no. 3, July 1998, pages 739 - 747, XP055350366
- SI-WON LEE ET AL.: 'Methylobacterium dankookense sp. nov., Isolated from Drinking Water' J. MICROBIOL. vol. 47, no. 6, December 2009, pages 716 - 720, XP055350364
- TAE HOO YI ET AL.: 'Sphingomonas humi sp. nov., Isolated from Soil' J. MICROBIOL. vol. 48, no. 2, April 2010, pages 165 - 169, XP055350361
- TAE-EUN CHOI ET AL.: 'Sphingomonas ginsenosidimutans sp. nov., with Ginsenoside Converting Activity' J. MICROBIOL. vol. 48, no. 6, December 2010, pages 760 - 766, XP055350360
- JOSEPH E. FITZGIBBON ET AL.: 'A sequence variant of Staphylococcus hominis with a high prevalence of oxacillin and fluoroquinolone resistance' RES. MICROBIOL. vol. 152, no. 9, November 2001, pages 805 - 810, XP055350358
- ROBERT B. SIMMONS ET AL.: 'The Occurrence and Persistence of Mixed Biofilms in Automobile Air Conditioning Systems' CURR. MICROBIOL. vol. 39, no. 3, 1999, pages 141 - 145, XP055260274

## Description

### BACKGROUND

### (a) Technical Field

The present invention relates to a method for screening an antimicrobial agent which is capable of controlling microorganisms that can cause offensive odor in an air-conditioning system and a method for removing offensive odor in an air-conditioning system.

### (b) Background Art

Clean air is recognized as an integral component in human health and well-being and offensive-smelling or polluted air hampers pleasant environment. For example, unsatisfactory indoor air quality under an airtight condition is caused by two important factors. One is the air pollutants generated from the structure (building or vehicle) itself constituting the airtight environment and the other is the odor generated by human activities or from materials taken in from outside.

An air-conditioning system, which is used in buildings, vehicles, trains, vessels, airplanes and the like, refers to a system designed to decrease indoor temperature and optimize the indoor environment for the purpose of conditioning the temperature, humidity, flow rate and cleanness of air. With improvement in the standard of living, the use of the air-conditioning system has been increasing gradually. Although there has been much improvement in the basic function of the air-conditioning system, a lot of problems still remain to be solved in the environmental aspect for indoor air quality.

The cause of the odor of the air-conditioning system, particularly an air conditioner, has been known to be the metabolites produced by molds and bacteria. However, types of molds and bacteria, and substances and quantity produced by the molds and bacteria have not been specifically identified yet.

In an air-conditioning system, all the air that has passed through a blower passes an evaporator core. During the heat exchange between a cold refrigerant and the air, condensation of water occurs on the surface of the evaporator core due to temperature difference. When the condensation of water continues, an environment favorable for inhabitation and proliferation of molds and bacteria is created on the evaporator core. If molds and bacteria proliferate on the evaporator core exposed to the external air, microbial volatile organic compounds (mVOCs) are produced as metabolites by the microorganisms. Thus, when the air that has passed through the evaporator core is blown indoors, the indoor may be exposed to offensive odor due to the volatile organic compounds produced by the molds and bacteria after long-term use.

After long-term use, the surface of the evaporator core is covered with a biofilm, which includes bacteria, cell clusters and extracellular polymeric substances (EPS). The EPS includes various components such as proteins, polysaccharides, polyuronic acids, nucleic acids, lipids, and the like. On the surface of the evaporator core, various bacteria and molds proliferate using the biofilm as nutrients and produce various microbial volatile organic compounds (mVOCs) as metabolites, which are known as one of the causes of the foul odor of the air conditioner.

Although various types of an air freshener are commercially available for removing such offensive odor, they do not fundamentally remove the molds and bacteria proliferating on the evaporator core but merely dilute the unpleasant odor temporarily. Also, the antimicrobial agents that are commercially available at present have not been developed to specifically act on particular molds or bacteria proliferating on the evaporator core but they have been used because they have antimicrobial effects against common pathogens. WO 02/27018 A2 relates to a novel method for identifying compounds capable of affecting a microbial biological activity, such as biofilm formation, development and dissolution. The method includes: (a) obtaining supernatant from a closed culture system that contains at least one type of microorganism; (b) exposing a target organism to the supernatant or an extract thereof; and (c) measuring the level of the biological activity. Simmons et al. report on the occurrence and persistence of mixed biofilms in automobile air conditioning systems (Current Microbiology, volume 39, issue 3, pages 8777-8793, 1999). Diekmann et al. report on microbial communities related to volatile organic compound emission in automobile air conditioning units (Applied Microbiology and Biotechnology, volume 97, issue 19, pages 8777-8793, 2013). WO 96/14882 A1 relates to an automative air-conditioning system comprising air-conditioning evaporator, heater, blower and duct characterized in which plastic material of housing, well-closed pad and lining pad of air-conditioning evaporator, door pad of heater housing, blowing-fan and lining pad of blower and duct are molded by mixing the antimicrobial compound selected from isothiazolines and oxybisphenoxarsines, and aluminum material of core of the said air-conditioning evaporator core is antimicrobially treated by coating hydrophilic coloring agent mixed with the antimicrobial composition of parachlorometa xylenol.

Accordingly, development of an antimicrobial agent which can create a pleasant indoor air environment by specifically inhibiting or preventing the proliferation of the molds and bacteria proliferating on the evaporator core and a method for removing offensive odor using the same is in urgent need.

The above description of the background art is intended only to improve understanding of the background of the present invention and should not be construed as recognizing that the above-described technologies are known to those having ordinary skill in the technical field to which the present invention pertains.

### SUMMARY

The inventors of the present invention have made efforts to find a method for effectively controlling microorganisms proliferating in an air-conditioning system and causing offensive odor. As a result, the inventors have successfully screened 12 species of microorganisms which proliferate in an air-conditioning system, form a biofilm and cause offensive odor and have confirmed that the offensive odor of the air-conditioning system can be significantly inhibited by controlling them. The invention is defined by the appended claims.

Disclosed is a method for screening an antimicrobial agent against a microorganism causing offensive odor in an air-conditioning system, which is selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri.*

Disclosed is also a microorganism causing offensive odor in an air-conditioning system.

Disclosed is also a method for inhibiting the growth of a microorganism causing offensive odor in an air-conditioning system, which includes coating or spraying an antimicrobial agent in an air-conditioning system.

Disclosed is also a method for removing offensive odor in an air-conditioning system, which includes separating or killing a microorganism causing offensive odor in an air-conditioning system.

Disclosed is also a method for removing offensive odor in an air-conditioning system, which includes inhibiting the growth of a microorganism causing offensive odor in an air-conditioning system.

Other features and aspects of the present invention will be apparent from the following detailed description, drawings and claims.

Disclosed is a method for screening an antimicrobial agent against a microorganism causing offensive odor in an air-conditioning system, which includes:
(a) preparing one or more microorganisms that cause offensive odor in an air-conditioning system and are selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri* or a culture thereof;
(b) contacting a sample to be analyzed with the microorganism;
(c) measuring the inhibition of the growth of the microorganism or the decrease in the generation of offensive odor; and
(d) determining that the sample has antimicrobial activity against the microorganism when the growth of the microorganism is inhibited or the generation of offensive odor is decreased.

Disclosed are successfully screened 12 species of microorganisms which proliferate in an air-conditioning system while forming a biofilm and cause offensive odor and have confirmed that the offensive odor of the air-conditioning system can be significantly inhibited by controlling them.

In the present disclosure, the term "air-conditioning system" refers to a system which maintains pleasant temperature, humidity, cleanness, flow, and the like. of air inside a space which is entirely or partially isolated from the external environment. As a specific example, the isolated space may be an indoor space which is entirely or partially isolated from outside, such as inside a building, vehicle, train, ship, airplane, and the like. As a specific example, the air-conditioning system may be an air conditioner.

In an air-conditioning system, all the air that has passed through a blower passes an evaporator core. On the surface of the evaporator core, an environment favorable for growth of microorganisms is created as condensation of water due to temperature difference continues. As a result, a biofilm is formed over time. The microorganisms metabolize various substances floating in the air indoors or outdoors as nutrients and produce various microbial volatile organic compounds (mVOCs) as metabolites, which give off offensive odor.

The biofilm is a group of living microorganisms enclosed in a membrane. The membrane protects the microorganisms from external environment and supplies nutrients. The membrane includes extracellular polymeric substances (EPS), which include various components such as proteins, polysaccharides, polyuronic acids, nucleic acids, lipids, and the like. On the surface of the evaporator core, a variety of microorganisms proliferate using them as nutrients and produce metabolites which give off offensive odor.

It is disclosed that microorganisms have been screened which cause offensive odor from the evaporator core and, through culturing, have isolated dominant strains from the microorganisms that form colonies. The dominant strains may be isolated and cultured according to various methods known in the related art and they may be screened, for example, based on dilution ratios or morphological characteristics such as the color, size, shape, and the like of the colonies.

The dominant microorganisms include microorganisms in the genus *Microbacterium, Methylobacterium, Sphingomonas* or *Staphylococcus,* preferably two species in the genus *Microbacterium* (*Microbacterium trichothecenolyticum* or *Microbacterium flavescens*), four species in the genus *Methylobacterium* (*Methylobacterium dankookense, Methylobacterium phyllosphaerae, Methylobacterium tardum* or *Methylobacterium radiotolerans*), four species in the genus *Sphingomonas* (*Sphingomonas dokdonensis, Sphingomonas ginsenosidimutans, Sphingomonas humi* or *Sphingomonas melonis*) or two species in the genus *Staphylococcus* (*Staphylococcus hominis subsp. hominis* or *Staphylococcus warneri*)*.*

These microorganisms were deposited on February 26, 2013 in the Korean Culture Center of Microorganisms and were given the following accession numbers: *Microbacterium trichothecenolyticum* HKMC-112 (accession number: KCCM11395P), *Microbacterium flavescens* HKMC-104 (accession number: KCCM11387P), *Methylobacterium dankookense* HKMC-101 (accession number: KCCM11384P), *Methylobacterium phyllosphaerae* HKMC-102 (accession number: KCCM11385P), *Methylobacterium tardum* HKMC-103 (accession number: KCCM11386P), *Methylobacterium radiotolerans* HKMC-111 (accession number: KCCM11394P), *Sphingomonas dokdonensis* HKMC-105 (accession number: KCCM11388P), *Sphingomonas ginsenosidimutans* HKMC-106 (accession number: KCCM11389P), *Sphingomonas humi* HKMC-107 (accession number: KCCM11390P), *Sphingomonas melonis* HKMC-108 (accession number: KCCM11391P), *Staphylococcus hominis subsp. hominis* HKMC-109 (accession number: KCCM11392P) and *Staphylococcus warneri* HKMC-110 (accession number: KCCM11393P).

The microorganisms causing offensive odor are industrially applicable for various purposes. For example, they may be used to develop a novel antimicrobial agent capable of inhibiting the growth of the microorganisms or to develop an air freshener for removing offensive odor by identifying the chemical properties of the metabolites of the microorganisms. In addition, they may be used to fundamentally remove the cause of offensive odor by providing an air-conditioning system with an environment where the microorganisms may not live.

The sample used in the method for screening an antimicrobial agent of the present disclosure is one to determine whether it has antimicrobial activity against the microorganisms. For example, if a particular sample has antimicrobial activity against *Methylobacterium dankookense,* the sample may be screened as an antimicrobial agent against *Methylobacterium dankookense.*

The antimicrobial agent screened by the screening method of the present disclosure may have antimicrobial activity against one or more selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri.* It may further have antimicrobial activity against other species of microorganisms.

For example, some antimicrobial agent may have antimicrobial activity against all the 12 species of microorganisms and another antimicrobial agent may have no antimicrobial activity at all against one or more of the species. In addition, the antimicrobial agent having antimicrobial activity against all the 12 species of microorganisms may have different antimicrobial activity against different microorganisms (see Tables 8 and 9).

The antimicrobial agent screened by the screening method of the present disclosure has antimicrobial activity against one or more selected from the group consisting of *Microbacterium trichothecenolyticum* HKMC-112 (accession number: KCCM11395P), *Microbacterium flavescens* HKMC-104 (accession number: KCCM11387P), *Methylobacterium dankookense* HKMC-101 (accession number: KCCM11384P), *Methylobacterium phyllosphaerae* HKMC-102 (accession number: KCCM11385P), *Methylobacterium tardum* HKMC-103 (accession number: KCCM11386P), *Methylobacterium radiotolerans* HKMC-111 (accession number: KCCM11394P), *Sphingomonas dokdonensis* HKMC-105 (accession number: KCCM11388P), *Sphingomonas ginsenosidimutans* HKMC-106 (accession number: KCCM11389P), *Sphingomonas humi* HKMC-107 (accession number: KCCM11390P), *Sphingomonas melonis* HKMC-108 (accession number: KCCM11391P), *Staphylococcus hominis subsp. hominis* HKMC-109 (accession number: KCCM11392P) and *Staphylococcus warneri* HKMC-110 (accession number: KCCM11393P).

Disclosed is that the sample to be screened includes a single compound, a mixture of compounds, an animal or plant extract, a biological agent containing genetic information such as a nucleotide, a polypeptide, and the like. and a mixture of compound and biological agent.

Disclosed is a microorganism causing offensive odor in an air-conditioning system.

The microorganism causing offensive odor may be *Microbacterium trichothecenolyticum,* more preferably *Microbacterium trichothecenolyticum* HKMC-112 deposited with an accession number KCCM11395P.

The microorganism causing offensive odor may be *Microbacterium flavescens,* more preferably *Microbacterium flavescens* HKMC-104 deposited with an accession number KCCM11387P.

The microorganism causing offensive odor may be *Methylobacterium dankookense,* more preferably *Methylobacterium dankookense* HKMC-101 deposited with an accession number KCCM11384P.

The microorganism causing offensive odor may be *Methylobacterium phyllosphaerae,* more preferably *Methylobacterium phyllosphaerae* HKMC-102 deposited with an accession number KCCM11385P.

The microorganism causing offensive odor may be *Methylobacterium tardum,* more preferably *Methylobacterium tardum* HKMC-103 deposited with an accession number KCCM11386P.

The microorganism causing offensive odor may be *Methylobacterium*
*radiotolerans,* more preferably *Methylobacterium radiotolerans* HKMC-111 deposited with an accession number KCCM11394P.

The microorganism causing offensive odor may be *Sphingomonas dokdonensis,* more preferably *Sphingomonas dokdonensis* HKMC-105 deposited with an accession number KCCM11388P.

The microorganism causing offensive odor may be *Sphingomonas ginsenosidimutans,* more preferably *Sphingomonas ginsenosidimutans* HKMC-106 deposited with an accession number KCCM11389P.

The microorganism causing offensive odor may be *Sphingomonas humi,* more preferably *Sphingomonas humi* HKMC-107 deposited with an accession number KCCM11390P.

The microorganism causing offensive odor may be *Sphingomonas melonis,* more preferably *Sphingomonas melonis* HKMC-108 deposited with an accession number KCCM11391P.

The microorganism causing offensive odor may be *Staphylococcus hominis subsp. hominis,* more preferably *Staphylococcus hominis subsp. hominis* HKMC-109 deposited with an accession number KCCM11392P.

The microorganism causing offensive odor may be *Staphylococcus warneri,* more preferably *Staphylococcus warneri* HKMC-110 deposited with an accession number KCCM11393P.

Disclosed is a method for inhibiting the growth of a microorganism causing offensive odor in an air-conditioning system, which includes coating or spraying the antimicrobial agent in an air-conditioning system.

The antimicrobial agent that may be used may be any antimicrobial agent which is determined or can be determined to have antimicrobial activity against one or more microorganisms selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri,* or against microorganisms comprising at least one of the above listed microorganism. The antimicrobial agent may be coated or sprayed in an air-conditioning system to inhibit the growth of one or more microorganisms causing offensive odor, which are selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri,* or the growth of microorganisms comprising at least one of the above listed microorganism. The antimicrobial agent may be coated or sprayed in various forms known in the art, such as gas, liquid, gel, suspension, and the like.

The coating or spraying may be performed partly or wholly on the inner surface or inner components of the air-conditioning system. Preferably, the coating or spraying may be performed on an evaporator core in the air-conditioning system. The coating or spraying may be performed after one or more microorganisms selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri,* or microorganism comprising at least one of the above listed microorganism has formed a biofilm or before one or more microorganisms selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri* or microorganism comprising at least one of the above listed microorganism forms a biofilm.

The evaporator core may be made of any material. Preferably, the evaporator core may be made of aluminum, an aluminum alloy, copper or a copper alloy.

Disclosed is a method for removing offensive odor in an air-conditioning system, which includes coating or spraying the antimicrobial agent in an air-conditioning system.

The coating or spraying may be performed to remove all or some of offensive odor or to prevent offensive odor before the offensive odor is generated.

Various microorganisms proliferate in an air-conditioning system. Those microorganisms can be largely classified into microorganisms causing offensive odor and microorganisms not causing offensive odor. Accordingly, if the antimicrobial agent acts specifically on the microorganisms causing offensive odor only or has inhibition activity against the growth of all or some of the dominant species of the microorganisms causing offensive odor, the offensive odor of the air-conditioning system may be improved partially or completely.

Disclosed is also a method for removing offensive odor in an air-conditioning system, which includes separating or killing one or more microorganisms causing offensive odor in an air-conditioning system, which is selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri.*

The microorganism or microorganism comprising at least one of the above listed microorganism may be separated or killed partially or completely via a physical, chemical or biological method. The physical method may be one artificially separating or killing the microorganism or microorganism comprising at least one of the above listed microorganism using a physical apparatus. The chemical method may be one separating or killing the microorganism or microorganism comprising at least one of the above listed microorganism using an antimicrobial agent or a sterilizer against the microorganism. The biological method may be one separating or killing the microorganism using a biological agent which is toxic to the microorganism or using another microorganism which competes with the microorganism for survival. However, the present disclosure is not limited by these examples.

Disclosed is a method for removing offensive odor in an air-conditioning system, which includes inhibiting the growth of one or more microorganisms causing offensive odor in an air-conditioning system, which is selected from the group consisting of *Microbacterium trichothecenolyticum, Microbacterium flavescens, Methylobacterium dankookense, Methylobacterium phyllosphaerae, Methylobacterium tardum, Methylobacterium radiotolerans, Sphingomonas dokdonensis, Sphingomonas ginsenosidimutans, Sphingomonas humi, Sphingomonas melonis, Staphylococcus hominis subsp. hominis* and *Staphylococcus warneri.*

The features and advantages of the present disclosure may be summarized as follows:
(i) The present invention provides a microorganism causing offensive odor in an air-conditioning system.
(ii) The present invention also provides a method for screening an antimicrobial agent which is capable of controlling the microorganism.
(iii) In addition, the present invention provides a method for removing offensive odor in an air-conditioning system by controlling the microorganism.
(iv) The microorganism causing offensive odor in an air-conditioning system of the present invention may be used to develop a novel antimicrobial or to develop an air-freshener for
   removing offensive odor by identifying the chemical properties of the metabolites of the microorganisms. In addition, it may be used to fundamentally remove the cause of offensive odor by providing an air-conditioning system with an environment where the microorganism may not live.

### BRIEF DESCRIPTION OF DRAWINGS

FIG. 1 shows a sample taken from an evaporator core of a used vehicle giving off offensive odor.
FIG. 2 shows a method of testing antimicrobial activity according to the present invention.
FIG. 3 shows a combination of dominant odorless microorganisms cultured on an aluminum fin, in which the aluminum is a material of an evaporator core.

### DETAILED DESCRIPTION

The present invention will be described in more detail through examples. The following examples are for illustrative purposes only and it will be apparent to those of ordinary skill in the art that the scope of this invention is not limited by the examples.

### Examples

### Example 1: Screening of dominant microorganisms causing offensive odor

### 1. Preparation of used vehicle giving off offensive odor and separation of air-conditioning system

In order to identify the cause of offensive odor generated in an airtight environment such as inside of a vehicle, air-conditioning systems were separated from 10 used vehicles giving off offensive odor in different seasons (winter: February-March, summer: June-July). Then, samples were taken from evaporator cores which were expected to have biofilms formed thereon by microorganisms causing offensive odor in the air-conditioning systems (Table 1).

**Table 1**

| No. | Mileage | Season |
|---|---|---|
| 1 | 89,000 km | Winter (February-March) |
| 2 | 70,000 km | |
| 3 | 10,300 km | |
| 4 | 37,100 km | |
| 5 | 149,970 km | |
| 6 | 35,000 km | Summer (June-July) |
| 7 | 28,000 km | |
| 8 | 42,000 km | |
| 9 | 110,000 km | |
| 10 | 90,000 km | |

### 2. Preparation of evaporator core sample

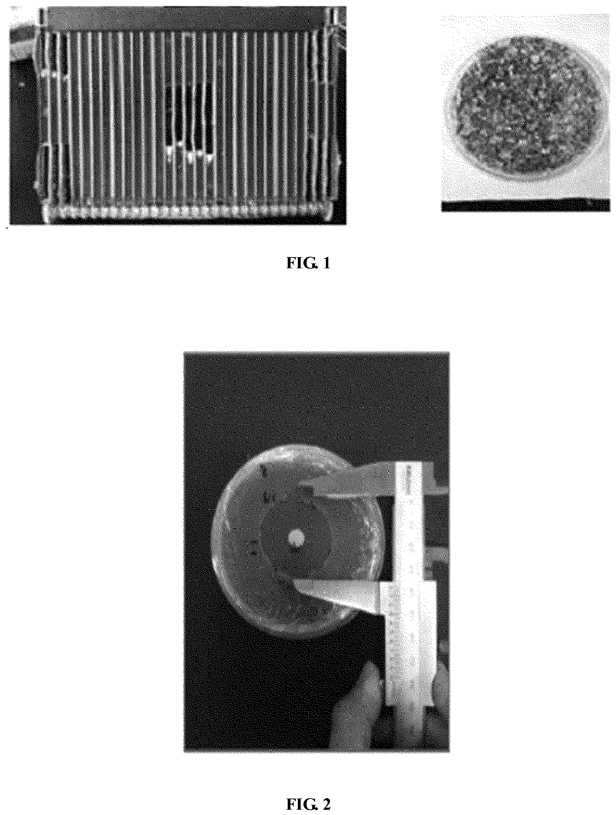

The evaporator cores separated from the used vehicles 1-10 giving off offensive odor were stored at a temperature of 4 °C before the evaporator core samples were used and was sealed in polyethylene bags. In order to isolate and culture microorganisms, 5 g of a sample was taken from random parts of each evaporator core, including front and rear surfaces, using sterilized long-nose pliers (FIG. 1).

### 3. Isolation of microorganisms

Microorganisms were isolated from the sample taken from the evaporator core as follows:
1) The sample taken from the evaporator core was mixed and put in a mixer.
2) 200 mL of sterilized 1x phosphate buffed saline (PBS) was added to the mixer.
3) The mixed sample and the PBS were mixed for 30 seconds.
4) The mixer was left on ice for 1 minute.
5) The steps 3) and 4) were repeated two more times.
6) The resulting suspension was centrifuged at a temperature of 4 °C for 3 minutes at 13000 rpm.
7) Only the supernatant was taken and transferred to a fresh tube.
8) The surface of the evaporator core from which the sample was taken was wiped several times with a sterilized cotton swab soaked with the supernatant.
9) The head of the cotton swab was put in the supernatant and then vortexed.
10) The precipitate obtained in the step 6) and the mixture obtained in the step 9) were mixed and used as an inoculation solution.

Microorganisms were physically isolated from the evaporator cores of the vehicles 1-10 through the steps 1)-10).

### 4. Isolation of microorganisms causing offensive odor and screening of dominant species

Aerobic heterotrophic bacteria usually called normal bacteria were isolated from the air conditioner by culturing on a heterotrophic plate. The normal bacteria were isolated by culturing at a temperature of 28-30 °C for 14 days using PTYG agar medium and R2A agar medium as complex nutrient media. The PTYG agar medium was prepared by adding 0.25 g of peptone (Difco), 0.25 g of triptone (Difco), 0.5 g of yeast extract (Difco), 0.5 g of glucose (Difco), 30 mg of MgSO₄ (Sigma), 3 mg of CaCl₂ (Sigma) and 15 g of Bacto agar (Difco) to 980 mL of distilled water and sterilizing at a temperature of 121 °C for 15 minutes under high pressure after adjusting pH to 7.0. The R2A agar medium was prepared by adding 0.5 g of yeast extract (Difco), 0.5 g of proteose peptone No.3 (Difco), 0.5 g of casamino acids (Difco), 0.5 g of dextrose (Difco), 0.5 g of soluble starch (Difco), 0.3 g of sodium pyruvate (Difco), 0.3 g of dipotassium sulfate (Difco), 0.05 g of magnesium sulfate (Difco) and 15 g of Bacto agar (Difco) to 980 mL of distilled water and sterilizing at a temperature of 121 °C for 15 minutes under high pressure after adjusting pH to 7.2. For isolation of non-dominant bacteria, antibiotic media were prepared by inoculating kanamycin, ampicillin or chloramphenicol at a temperature of 50 °C after filter-sterilizing the media to a concentration of 100 ppm.

Dominant strains were isolated and cultured based on dilution ratios or morphological characteristics such as the color, size, shape, and the like of the colonies as follows.
1) Molds and bacteria were separated from an isolated-culture media.
2) The bacteria exhibiting different morphologies were separated by inoculating to complex media using a loop.
3) From the inoculated media, the bacterial culture showing the best growth state was selected and subcultured.
4) The molds were inoculated to complex media after removing the hypha end portions using a scalpel.
5) From the inoculated media, the mold culture showing the best growth state was selected and subcultured.

### 5. Identification of dominant microorganisms

For accurate identification of the isolated microorganisms, 16s rRNA analysis was performed as follows.

### a) Fingerprinting based on REP-PCR pattern analysis

REP-PCR is a molecular biological fingerprinting technique for structural analysis of bacterial chromosomes, which allows distinction of each bacterial strain. Genetic characteristics were analyzed by REP-PCR as follows.

### (1) Cell lysis

1) 2.5 µL of a Lyse-N-Go PCR reagent (Thermo) was added to a PCR tube.
2) A colony was pipetted onto the tube on a clean bench. During the pipetting, caution was made such that the resulting solution did not become turbid.
3) Culturing was performed on a PCR machine according to the manufacturer's instructions.

### (2) PCR reaction

Using a PCR reagent prepared as described in Table 2, PCR amplification was carried out by conducting pre-denaturation at a temperature of 93 °C for 7 minutes and repeating 33 cycles of denaturation at a temperature of 92 °C for 1 minute, annealing at a temperature of 51.5 °C for 1 minute and extension at a temperature of 65 °C for 8 minutes, as described in Table 3.

**Table 2**

| | | |
|---|---|---|
| ① | dNTP (2.5 mM each) | 12.5 µL |
| ② | Gitschier buffer | 5.0 µL |
| ③ | DMSO (100%) | 2.5 µL |
| ④ | Autoclaved 3° D.W. | 0.3 µL |
| ⑤ | BOXA1R primer (50 pmole/µL) 5'CTACGGCAAGGCGACGCTGACG | 1.0 µL |
| ⑥ | BSA (10 mg/mL) | 0.4 µL |
| ⑦ | Bacterial DNA | 2.5 µL |
| ⑧ | Taq polymerase (Roche) (5 U/µL) | 0.8 µL |

**Table 3**

| | | |
|---|---|---|
| Step 1 | 93 °C | 7 min |
| Step 2 | 92 °C | 1 min |
| Step 3 | 51.5 °C | 1 min |
| Step 4 | 65 °C | 8 min |
| Step 5 | Steps 2, 3 & 4: additional 33 cycles | |
| Step 6 | 65 °C | 16 min |
| Step 7 | 4 °C | |

### (3) Gel electrophoresis

The PCR-amplified DNA fragments were loaded onto 1.2-1.5% agarose gel supplemented with EtBr after mixing a 6x dye with the sample at a ratio of 1:5. Since most PCR products were in the range of 100-1000 bp, they were loaded tougher with 100 bp ladders. Then, electrophoresis was carried out as slowly as possible (50 V) such that bromophenol blue and xylene cyanol dyes moved halfway of the entire gel. The strains exhibiting the same DNA pattern on the gel were regarded as the same strains.

### b) Identification of dominant bacteria from air conditioner based on 16S rRNA gene analysis

The 16S ribosomal ribonucleic acid (rRNA) gene is used for genetic identification of bacteria. The bacteria differentiated by REP-PCR can be identified in the levels of genus and species.

### (1) Cell lysis

1) 5 µL of a Lyse-N-Go PCR reagent (Thermo) was added to a PCR tube.
2) A colony was pipetted onto the tube on a clean bench. The pipetting was performed such that the resulting solution became slightly turbid.
3) Cell lysis was performed on a PCR machine according to the manufacturer's instructions (Table 4).

**Table 4: Lysis program**

| Cycle | Temperature (°C) | Time (seconds) |
|---|---|---|
| 1 | 65 | 30 |
| 2 | 8 | 30 |
| 3 | 65 | 90 |
| 4 | 97 | 180 |
| 5 | 8 | 60 |
| 6 | 65 | 180 |

### (2) 16S rRNA PCR

A mixture (44.5 µL) of the solutions described in the following Table 5 below, except for DNA and Taq, was added to the lysis solution described above (total volume of 50 µL in Table 5). Subsequently, PCR amplification was carried out by conducting pre-denaturation at a temperature of 94 °C for 5 minutes and repeating 29 cycles of denaturation at a temperature of 94 °C for 1 minute, annealing at a temperature of 55 °C for 1 minute and extension at a temperature of 72 °C for 1 minute and 30 seconds, as described in Table 6.

**Table 5**

| | |
|---|---|
| Autoclaved 3° D.W. | 22 µL |
| 10x buffer (Roche) | 5 µL |
| dNTP (Roche, 2.5 mM) | 5 µL |
| DMSO | 5 µL |
| BSA (10 mg/mL) | 2.5 µL |
| 27mf (20 pmole/µL) | 2.5 µL |
| 1492r (20 pmole/µL) | 2.5 µL |
| DNA | 5 µL |
| Taq (Roche) | 0.5 µL |

**Table 6**

| | | |
|---|---|---|
| Step 1 | 94 °C | 5 min |
| Step 2 | 94 °C | 1 min |
| Step 3 | 55 °C | 1 min |
| Step 4 | 72 °C | 1 min 30 sec |
| Step 5 | Go to step 2: additional 29 cycles | |
| Step 6 | 72 °C | 10 min |
| Step 7 | 4 °C | hold |

### (3) PCR purification

The 16S rRNA PCR products were purified using a QIAquick PCR purification kit as follows.
1) The PCR products were added to a 5x PB buffer.
2) The resulting solution was transferred to a QIAquick column.
3) For DNA binding, the solution was centrifuged for 1 minute, and then filtered solution was removed.
4) For washing, 750 µL of PE buffer was added to the QIAquick column and centrifugation was performed for 1 minute, and then filtered solution was removed.
5) Centrifugation was performed for 1 minute.
6) The QIAquick column was transferred to a new tube.
7) For DNA extraction, 30 µL of EB buffer was added and the resulting solution was allowed to stand for 1 minute.
8) After performing centrifugation for 1 minute, the DNA dissolved in EB was collected in a tube.

In order to observe whether the isolated microorganisms give off offensive odor, sensory evaluation was performed as follows.
1) The isolated microorganisms were inoculated to a liquid nutrient medium.
2) Culturing was performed at a temperature of 28 °C for 5-7 days.
3) 100 µL of the microorganisms cultured in the liquid medium were inoculated to a solid nutrient medium.
4) The inoculated microorganisms were spread uniformly using a spreader.
5) The microorganisms were cultured on a sealed Petri dish at a temperature of 28 °C for 10 days.

The dominant microorganisms causing offensive odor were screened based on the average of the sensory evaluation by 7 panelists on a 5-point scale. A total of 12 dominant species were identified through the 16S rRNA analysis, as shown in the following Table 7, and they were deposited in the Korean Culture Center of Microorganisms on February 26, 2013.

**Table 7: Accession numbers of 12 dominant microorganisms causing offensive odor**

| No. | Identification No. | Name | Accession No. |
|---|---|---|---|
| 1 | HKMC-101 | *Methylobacterium dankookense* | KCCM11384P |
| 2 | HKMC-102 | *Methylobacterium phyllosphaerae* | KCCM11385P |
| 3 | HKMC-103 | *Methylobacterium tardum* | KCCM11386P |
| 4 | HKMC-104 | *Microbacterium flavescens* | KCCM11387P |
| 5 | HKMC-105 | *Sphingomonas dokdonensis* | KCCM11388P |
| 6 | HKMC-106 | *Sphingomonas ginsenosidimutans* | KCCM11389P |
| 7 | HKMC-107 | *Sphingomonas humi* | KCCM 11390P |
| 8 | HKMC-108 | *Sphingomonas melonis* | KCCM11391P |
| 9 | HKMC-109 | *Staphylococcus hominis subsp. hominis* | KCCM11392P |
| 10 | HKMC-110 | *Staphylococcus warneri* | KCCM11393P |
| 11 | HKMC-111 | *Methylobacterium radiotolerans* | KCCM11394P |
| 12 | HKMC-112 | *Microbacterium trichothecenolyticum* | KCCM11395P |

### Example 2: Evaluation of antimicrobial activity of antimicrobial agent against screened microorganisms causing offensive odor

### 1. Experimental procedure

The antimicrobial activity of various commercially available antimicrobial agents against the dominant microorganisms screened in Example 1 was evaluated. The tested antimicrobial agents are as follows:
Antimicrobial agent A: fabric deodorizer purchased from P&G Korea.
Antimicrobial agent B: hand sanitizer purchased from Paru Corporation.
Antimicrobial agent C: mass-produced antimicrobial agent containing 45-50% methyl alcohol, 1-5% chromium sulfate (CAS 10101-53-8), 1-5% bromine and water.
Antimicrobial agent D: cationic antimicrobial agent (Parkerizing, Japan).
Antimicrobial agent E: isothiazolinone-based antimicrobial agent containing methylisothiazolinone (CAS 26172-55-4), bronopol (CAS 52-51-7), and the like. (Parkerizing, Japan).

The antimicrobial activity was evaluated as follows:
1) Sterilized filter paper was prepared.
2) Five antimicrobial agents were prepared (control group: not treated with an antimicrobial agent, test groups: treated with antimicrobial agent A, antimicrobial agent B, antimicrobial agent C, antimicrobial agent D and antimicrobial agent E).
3) Each antimicrobial agent was added to the filter paper.
4) Each microorganism causing offensive odor was coated on a nutrient medium.
5) The filter paper to which the antimicrobial agent was added was placed on the nutrient medium on which the microorganism causing offensive odor was coated.
6) The microorganism was cultured at a temperature of 28-30 °C for 5 days.
7) The growth inhibition zone was measured.

The diameter of the growth inhibition zone was measured using a vernier caliper as shown in FIG. 2.

### 2. Experimental result

The result of measuring the diameter of the growth inhibition zone for the 12 species of microorganisms causing offensive odor is shown in Table 8.

**Table 8: Diameter of growth inhibition zone (unit: mm)**

| No. | Microorganism (Deposition Name) | Antimicrobial agent | | | | | |
|---|---|---|---|---|---|---|---|
| | | None | A | B | C | D | E |
| 1 | *Methylobacterium dankookense* HKMC-101 | 0 | 18.3 | 22.3 | 27.3 | 29.5 | 36.1 |
| 2 | *Methylobacterium phyllosphaerae* HKMC-102 | 0 | 18.3 | 11.6 | 35.3 | 47.3 | 45.3 |
| 3 | *Methylobacterium tardum* HKMC-103 | 0 | 0 | 0 | 0 | 43 | 38.3 |
| 4 | *Microbacterium flavescens* HKMC-104 | 0 | 19.6 | 14 | 28 | 19.1 | 33.9 |
| 5 | *Sphingomonas dokdonensis* HKMC-105 | 0 | 15 | 11.6 | 21.7 | 16.2 | 39.1 |
| 6 | *Sphingomonas ginsenosidimutans* HKMC-106 | 0 | 0 | 11.3 | 17.6 | 21.8 | 31.1 |
| 7 | *Sphingomonas humi* HKMC-107 | 0 | 11.6 | 14.3 | 22.5 | 19.1 | 36.1 |
| 8 | *Sphingomonas melonis* HKMC-108 | 0 | 17 | 11 | 31.6 | 22.6 | 37.3 |
| 9 | *Staphylococcus hominis subsp. hominis* HKMC-109 | 0 | 0 | 0 | 25.6 | 28.6 | 54 |
| 10 | *Staphylococcus warneri* HKMC-110 | 0 | 0 | 0 | 33.3 | 17 | 38.6 |
| 11 | *Methylobacterium radiotolerans* HKMC-111 | 0 | 17.6 | 0 | 23.3 | 28.1 | 31 |
| 12 | *Microbacterium trichothecenolyticum* HKMC-112 | 0 | 19 | 18.3 | 20.6 | 18.5 | 32.3 |

The antimicrobial activity of the cationic antimicrobial agent (antimicrobial agent D) and the isothiazolinone-based antimicrobial agent (antimicrobial agent E) were compared with that of the mass-produced antimicrobial agent (antimicrobial agent C), and the results are shown in Table 9 below.

**Table 9: Antimicrobial activity of cationic antimicrobial agent and isothiazolinone-based antimicrobial agent compared with that of mass-produced antimicrobial agent**

| No. | Microorganism (Deposition Name) | Antimicrobial agent | |
|---|---|---|---|
| | | D | E |
| 1 | *Methylobacterium dankookense* HKMC-101 | 8.1% | 32.2% |
| 2 | *Methylobacterium phyllosphaerae* HKMC-102 | 34% | 28.3% |
| 3 | *Methylobacterium tardum* HKMC-103 | 43% | 38.3% |
| 4 | *Microbacteriumflavescens* HKMC-104 | -31.8% | 21% |
| 5 | *Sphingomonas dokdonensis* HKMC-105 | -25.3% | 80.1% |
| 6 | *Sphingomonas ginsenosidimutans* HKMC-106 | 23.8% | 76.7% |
| 7 | *Sphingomonas humi* HKMC-107 | -15.1% | 60.4% |
| 8 | *Sphingomonas melonis* HKMC-108 | -28.5% | 18% |
| 9 | *Staphylococcus hominis subsp. hominis* HKMC-109 | 11.7% | 110.9% |
| 10 | *Staphylococcus warneri* HKMC-110 | -48.9% | 15.9% |
| 11 | *Methylobacterium radiotolerans* HKMC-111 | 20.6% | 33% |
| 12 | *Microbacterium trichothecenolyticum* HKMC-112 | -10.2% | 56.8% |

As shown in the above Tables 8 and 9, the antimicrobial agent A exhibited no antimicrobial activity against *Methylobacterium tardum, Sphingomonas ginsenosidimutans, Staphylococcus hominis subsp. hominis* and *Staphylococcus warneri* at all. Although the antimicrobial agent B showed antimicrobial activity against *Sphingomonas ginsenosidimutans* unlike the antimicrobial agent A, it showed no antimicrobial activity against *Methylobacterium radiotolerans.*

In addition, the antimicrobial agent C showed no antimicrobial activity against *Methylobacterium tardum.* In contrast, the antimicrobial agents D and E showed antimicrobial activity against all the 12 species of microorganisms. However, the antimicrobial agent D showed lower antimicrobial activity against *Microbacterium flavescens, Sphingomonas dokdonensis, Sphingomonas humi, Sphingomonas melonis, Staphylococcus warneri* and *Microbacterium trichothecenolyticum* as compared to that of the antimicrobial agent C. For the microorganisms belonging to the same genus *Methylobacterium,* the antimicrobial agent E showed more specific antimicrobial activity against *Methylobacterium dankookense* and *Methylobacterium radiotolerans* as compared to the antimicrobial agent D, and the antimicrobial agent D showed more specific antimicrobial activity against *Methylobacterium phyllosphaerae* and *Methylobacterium tardum* as compared to the antimicrobial agent E. That is to say, the antimicrobial agents showed different antimicrobial activity against different microorganisms belonging to the same genus.

### Example 3: Evaluation of odor of evaporator core with microorganism causing offensive odor removed

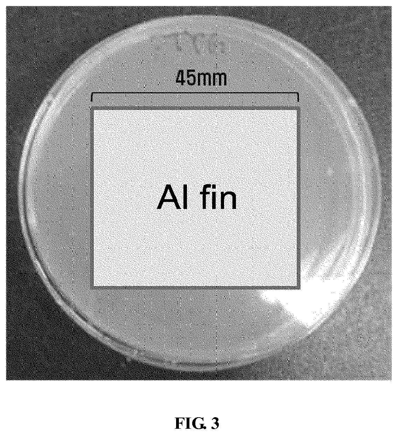

In order to prepare an evaporator core from which microorganisms causing offensive odor were removed or separated, combinations of odorless microorganisms excluding the microorganisms causing offensive odor of Example 1 from the dominant microorganisms inhabiting the evaporator core were cultured on an aluminum fin, in which the aluminum was a material of an evaporator core (Table 10, FIG. 3).

The dominant species which formed colonies on the evaporator core but did not cause offensive odor were selected as the odorless microorganisms and cultured as follows.
1) The isolated odorless microorganisms were inoculated to a liquid R2A medium.
2) The microorganisms were cultured at a temperature of 28 °C for 5-7 days.
3) An aluminum fin sterilized at a temperature of 121 °C for 20 minutes under high pressure was prepared.
4) The surface of the aluminum fin was uniformly coated with each antimicrobial agent.
5) The coated aluminum fin was placed on a Petri dish.
6) 1 mL of the odorless microorganism culture was centrifuged and the supernatant was discarded.
7) After adding 1 mL of sterilized 1 x PBS, the mixture was centrifuged again.
8) The step 7) was repeated two times.
9) 100 µL of the odorless microorganism culture washed with PBS was dropped onto the center of the aluminum fin.
10) The aluminum fin was dried at room temperature.
11) After sealing the Petri dish, the odorless microorganisms were cultured at a temperature of 28 °C for 1 month.

As a result, all the combinations described in the following Table 10 were found not to cause offensive odor.

**Table 10: Odor of microorganisms inhabiting evaporator core having microorganisms causing offensive odor removed**

| Combination No. | Microorganisms | Odor after culturing for 1 month |
|---|---|---|
| 1 | *Methylobacterium brachiatum* | Odorless |
| 2 | *Methylobacterium platani* | Odorless |
| 3 | *Methylobacterium aquaticum* + *Methylobacterium platani* | Odorless |
| 4 | *Methylobacterium platani* + *Methylobacterium brachiatum* | Odorless |
| 5 | *Methylobacterium aquaticum* + *Methylobacterium platani* + *Methylobacterium brachiatum* | Odorless |
| 6 | *Methylobacterium aquaticum* + *Methylobacterium platani* + *Methylobacterium brachiatum* + *Acinetobacter johnsonii* | Odorless |
| 7 | *Methylobacterium aquaticum* + *Methylobacterium platani* + *Methylobacterium brachiatum* + *Bacillus vietnamensis* | Odorless |
| 8 | *Methylobacterium aquaticum* + *Methylobacterium platani* + *Methylobacterium brachiatum* + *Brevibacillus invocatus* | Odorless |
| 9 | *Methylobacterium aquaticum* + *Methylobacterium platani* + *Methylobacterium brachiatum* + *Deinococcus ficus* | Odorless |
| 10 | *Methylobacterium aquaticum* + *Methylobacterium platani* + *Methylobacterium brachiatum* + *Leifsonia soli* | Odorless |
| 11 | *Methylobacterium aquaticum* + *Methylobacterium platani* + *Methylobacterium brachiatum* + *Methylobacterium komagatae* | Odorless |
| 12 | *Methylobacterium aquaticum* + *Methylobacterium platani* + *Methylobacterium brachiatum* + *Pseudomonas nitroreducens* | Odorless |
| 13 | *Methylobacterium aquaticum* + *Methylobacterium platani* + *Methylobacterium brachiatum* + *Sphingomonas aquatilis* | Odorless |
| 14 | *Sphingomonas aquatilis* + *Brevibacillus invocatus* | Odorless |
| 15 | *Leifsonia soli* + *Methylobacterium komagatae* | Odorless |
| 16 | *Acinetobacter johnsonii* + *Sphingomonas aquatilis* + *Methylobacterium komagatae* | Odorless |
| 17 | *Pseudomonas nitroreducens* | Odorless |
| 18 | *Acinetobacter johnsonii* + *Pseudomonas nitroreducens* | Odorless |
| 19 | *Brevibacillus invocatus* + *Acinetobacter johnsonii* + *Pseudomonas nitroreducens* | Odorless |
| 20 | *Leifsonia soli* + *Pseudomonas nitroreducens* | Odorless |
| 21 | *Brevibacillus invocatus* + *Sphingomonas aquatilis* + *Pseudomonas nitroreducens* | Odorless |
| 22 | *Acinetobacter johnsonii* + *Sphingomonas aquatilis* + *Pseudomonas nitroreducens* | Odorless |
| 23 | *Methylobacterium aquaticum* + *Methylobacterium komagatae* + *Bacillus vietnamensis* + *Deinococcus ficus* | Odorless |
| 24 | *Methylobacterium aquaticum* + *Methylobacterium komagatae* + *Curtobacterium flaccumfaciens* + *Deinococcus apachensis* + *Bacillus subtilis subsp. subtilis* | Odorless |
| 25 | *Methylobacterium aquaticum* + *Methylobacterium komagatae* + *Spirosoma linguale* +*Sphingomonas dokdonensis* + *Leifsonia soli* | Odorless |

From Table 10, it can be seen that the offensive odor generated from an air-conditioning system can be significantly removed by chemically or physically removing the microorganisms causing offensive odor from the microorganisms inhabiting the air-conditioning system and providing combinations of microorganisms not causing offensive odor.

The microorganism causing offensive odor in an air-conditioning system of the present invention is industrially applicable for various purposes. For example, they may be used to develop a novel antimicrobial agent capable of inhibiting the growth of the microorganisms or to develop an air freshener for removing offensive odor by elucidating the chemical properties of the metabolites of the microorganisms. In addition, they may be used to fundamentally remove the cause of offensive odor by providing an air-conditioning system with an environment where the microorganisms may not live.

## Claims

1. A method for screening an antimicrobial agent against a microorganism causing offensive odor in an air-conditioning system, which comprises:
(a) preparing one or more microorganisms that cause offensive odor in an air-conditioning system and are selected from the group consisting of *Microbacterium trichothecenolyticum* HKMC-112 (accession number: KCCM11395P), *Microbacterium flavescens* HKMC-104 (accession number: KCCM11387P), *Methylobacterium dankookense* HKMC-101 (accession number: KCCM11384P), *Methylobacterium phyllosphaerae* HKMC-102 (accession number: KCCM11385P), *Methylobacterium tardum* HKMC-103 (accession number: KCCM11386P), *Methylobacterium radiotolerans* HKMC-111 (accession number: KCCM11394P), *Sphingomonas dokdonensis* HKMC-105 (accession number: KCCM11388P), *Sphingomonas ginsenosidimutans* HKMC-106 (accession number: KCCM11389P), *Sphingomonas humi* HKMC-107 (accession number: KCCM11390P), *Sphingomonas melonis* HKMC-108 (accession number: KCCM11391P), *Staphylococcus hominis subsp. hominis* HKMC-109 (accession number: KCCM11392P), *Staphylococcus warneri* HKMC-110 (accession number: KCCM11393P) or a culture thereof;
(b) contacting a sample to be analyzed with the microorganism;
(c) measuring an inhibition of the growth of the microorganism or a decrease in the generation of offensive odor; and
(d) determining that the sample has antimicrobial activity against the microorganism if the growth of the microorganism is inhibited or the generation of offensive odor is decreased.

2. The screening method according to claim 1, wherein the air-conditioning system is an air conditioner.

3. The screening method according to claim 1 or 2, wherein the microorganism causes offensive odor by forming a biofilm on an evaporator core in the air-conditioning system.

4. The screening method according to claim 3, wherein the evaporator core is made of aluminum, an aluminum alloy, copper or a copper alloy.

5. A microorganism causing offensive odor used in an air-conditioning system, the microorganism being selected from the group consisting of *Microbacterium flavescens* HKMC-104 (accession number: KCCM11387P), *Methylobacterium dankookense* HKMC-101 (accession number: KCCM11384P), *Methylobacterium phyllosphaerae* HKMC-102 (accession number: KCCM11385P), *Methylobacterium tardum* HKMC-103 (accession number: KCCM11386P), *Methylobacterium radiotolerans* HKMC-111 (accession number: KCCM11394P), *Sphingomonas dokdonensis* HKMC-105 (accession number: KCCM11388P), *Sphingomonas ginsenosidimutans* HKMC-106 (accession number: KCCM11389P), *Sphingomonas humi* HKMC-107 (accession number: KCCM11390P), *Sphingomonas melonis* HKMC-108 (accession number: KCCM11391P), *Staphylococcus hominis subsp. hominis* HKMC-109 (accession number: KCCM11392P), and *Staphylococcus warneri* HKMC-110 (accession number: KCCM11393P).

6. A method for removing offensive odor in an air-conditioning system, which comprises separating or killing one or more microorganisms causing offensive odor in an air-conditioning system, which is selected from the group consisting of *Microbacterium flavescens* HKMC-104 (accession number: KCCM11387P), *Methylobacterium dankookense* HKMC-101 (accession number: KCCM11384P), *Methylobacterium phyllosphaerae* HKMC-102 (accession number: KCCM11385P), *Methylobacterium tardum* HKMC-103 (accession number: KCCM11386P), *Methylobacterium radiotolerans* HKMC-111 (accession number: KCCM11394P), *Sphingomonas dokdonensis* HKMC-105 (accession number: KCCM11388P), *Sphingomonas ginsenosidimutans* HKMC-106 (accession number: KCCM11389P), *Sphingomonas humi* HKMC-107 (accession number: KCCM11390P), *Sphingomonas melonis* HKMC-108 (accession number: KCCM11391P), *Staphylococcus hominis subsp. hominis* HKMC-109 (accession number: KCCM11392P), and *Staphylococcus warneri* HKMC-110 (accession number: KCCM11393P).

7. A method for removing offensive odor in an air-conditioning system, which comprises inhibiting a growth of one or more microorganisms causing offensive odor in an air-conditioning system, which is selected from the group consisting of *Microbacterium flavescens* HKMC-104 (accession number: KCCM11387P), *Methylobacterium dankookense* HKMC-101 (accession number: KCCM11384P), *Methylobacterium phyllosphaerae* HKMC-102 (accession number: KCCM11385P), *Methylobacterium tardum* HKMC-103 (accession number: KCCM11386P), *Methylobacterium radiotolerans* HKMC-111 (accession number: KCCM11394P), *Sphingomonas dokdonensis* HKMC-105 (accession number: KCCM11388P), *Sphingomonas ginsenosidimutans* HKMC-106 (accession number: KCCM11389P), *Sphingomonas humi* HKMC-107 (accession number: KCCM11390P), *Sphingomonas melonis* HKMC-108 (accession number: KCCM11391P), *Staphylococcus hominis subsp. hominis* HKMC-109 (accession number: KCCM11392P), and *Staphylococcus warneri* HKMC-110 (accession number: KCCM11393P).

## Patentansprüche

1. Screening-Verfahren für einen antimikrobiellen Wirkstoff gegen einen Mikroorganismus, der einen unangenehmen Geruch in einer Klimaanlage verursacht, das Folgendes umfasst:
(a) Anfertigen eines oder mehrerer Mikroorganismen, die einen unangenehmen Geruch in einer Klimaanlage verursachen und ausgewählt sind aus der Gruppe bestehend aus *Microbacterium trichothecenolyticum* HKMC-112 (Akzessionsnummer: KCCM11395P), *Microbacterium flavescens* HKMC-104 (Akzessionsnummer: KCCM11387P), *Methylobacterium dankookense* HKMC-101 (Akzessionsnummer: KCCM 11384P), *Methylobacterium phyllosphaerae* HKMC-102 (Akzessionsnummer: KCCM11385P), *Methylobacterium tardum* HKMC-103 (Akzessionsnummer: KCCM11386P), *Methylobacterium radiotolerans* HKMC-111 (Akzessionsnummer: KCCM11394P), *Sphingomonas dokdonensis* HKMC-105 (Akzessionsnummer: KCCM11388P), *Sphingomonas ginsenosidimutans* HKMC-106 (Akzessionsnummer: KCCM11389P), *Sphingomonas humi* HKMC-107 (Akzessionsnummer: KCCM11390P), *Sphingomonas melonis* HKMC-108 (Akzessionsnummer: KCCM11391P), *Staphylococcus hominis subsp. hominis* HKMC-109 (Akzessionsnummer: KCCM11392P), *Staphylococcus warneri* HKMC-110 (Akzessionsnummer: KCCM11393P) oder einer Kultur davon;
(b) Inkontaktbringen einer zu analysierenden Probe mit dem Mikroorganismus;
(c) Messen einer Hemmung des Wachstums des Mikroorganismus oder einer Abnahme der Bildung eines unangenehmen Geruchs; und
(d) Feststellen, dass die Probe eine antimikrobielle Aktivität gegen den Mikroorganismus aufweist, falls das Wachstum des Mikroorganismus gehemmt ist oder die Bildung eines unangenehmen Geruchs abgenommen hat.

2. Screening-Verfahren nach Anspruch 1, wobei die Klimaanlage ein Luftkonditionierer ist.

3. Screening-Verfahren nach Anspruch 1 oder 2, wobei der Mikroorganismus einen unangenehmen Geruch durch Bilden eines Biofilms auf einem Verdampferkern in der Klimaanlage verursacht.

4. Screening-Verfahren nach Anspruch 3, wobei der Verdampferkern aus Aluminium, einer Aluminiumlegierung, Kupfer oder einer Kupferlegierung besteht.

5. Mikroorganismus, der einen unangenehmen Geruch in einer Klimaanlage verursacht, wobei der Mikroorganismus ausgewählt ist aus der Gruppe bestehend aus *Microbacterium flavescens* HKMC-104 (Akzessionsnummer: KCCM 11387P), *Methylobacterium dankookense* HKMC-101 (Akzessionsnummer: KCCM 11384P), *Methylobacterium phyllosphaerae* HKMC-102 (Akzessionsnummer: KCCM 11385P), *Methylobacterium tardum* HKMC-103 (Akzessionsnummer: KCCMI1386P), *Methylobacterium radiotolerans* HKMC-III (Akzessionsnummer: KCCM 11394P), *Sphingomonas dokdonensis* HKMC-105 (Akzessionsnummer: KCCM 11388P), *Sphingomonas ginsenosidimutans* HKMC-106 (Akzessionsnummer: KCCM11389P), *Sphingomonas humi* HKMC-107 (Akzessionsnummer: KCCM11390P), *Sphingomonas melonis* HKMC-108 (Akzessionsnummer: KCCM11391P), *Staphylococcus hominis subsp. hominis* HKMC-109 (Akzessionsnummer: KCCM11392P), und *Staphylococcus warneri* (Akzessionsnummer: KCCMI 1393P).

6. Verfahren zum Beseitigen eines unangenehmen Geruchs in einer Klimaanlage, das das Abtrennen oder Abtöten eines oder mehrerer einen unangenehmen Geruch in einer Klimaanlage verursachenden Mikroorganismen umfasst, die ausgewählt sind aus der Gruppe bestehend aus *Microbacterium flavescens* HKMC-104 (Akzessionsnummer: KCCM11387P), *Methylobacterium dankookense* HKMC-101 (Akzessionsnummer: KCCMI 1384P), *Methylobacterium phyllosphaerae* HKMC-102 (Akzessionsnummer: KCCM11385P), *Methylobacterium tardum* HKMC-103 (Akzessionsnummer: KCCM11386P), *Methylobacterium radiotolerans* HKMC-111 (Akzessionsnummer: KCCM11394P), *Sphingomonas dokdonensis* HKMC-105 (Akzessionsnummer: KCCM11388P), *Sphingomonas ginsenosidimutans* HKMC-146 (Akzessionsnummer: KCCM11389P), *Sphingomonas humi* HKMC-107 (Akzessionsnummer: KCCM11390P), *Sphingomonas melonis* HKMC-108 (Akzessionsnummer: KCCM11391P), *Staphylococcus hominis subsp. hominis* HKMC-109 (Akzessionsnummer: KCCM11392P), und *Staphylococcus warneri* HKMC-110 (Akzessionsnummer: KCCM11393P).

7. Verfahren zum Beseitigen eines unangenehmen Geruchs in einer Klimaanlage, welches das Hemmen eines Wachstums einer oder mehrerer einen unangenehmen Geruch in einer Klimaanlage verursachenden Mikroorganismen umfasst, die ausgewählt sind aus der Gruppe bestehend aus *Microbacterium flavescens* HKMC-104 (Akzessionsnummer: KCCM11387P), *Methylobacterium dankookense* HKMC-101 (Akzessionsnummer: KCCM 11384P), *Methylobacterium phyllosphaerae* HKMC-102 (Akzessionsnummer: KCCM11385P), *Methylobacterium tardum* HKMC-103 (Akzessionsnummer: KCCMI1386P), *Methylobacterium radiotolerans* HKMC-III (Akzessionsnummer: KCCM11394P), Sphingomonas dokdonensis HKMC-105 (Akzessionsnummer: KCCM11388P), *Sphingomonas ginsenosidimutans* HKMC-106 (Akzessionsnummer: KCCM11389P), *Sphingomonas humi* HKMC-107 (Akzessionsnummer: KCCM11390P), *Sphingomonas melonis* HKMC-148 (Akzessionsnummer: KCCMI1391P), *Staphylococcus hominis subsp. hominis* HKMC-109 (Akzessionsnummer: KCCM11392P), und *Staphylococcus warneri* HKMC-110 (Akzessionsnummer: KCCM11393P).

## Revendications

1. Procédé de ciblage d'un agent antimicrobien contre un microorganisme provoquant une odeur déplaisante dans un système de conditionnement d'air, qui comprend :
(a) la préparation d'un ou de plusieurs microorganismes qui provoquent une odeur déplaisante dans un système de conditionnement d'air et sont choisis dans le groupe constitué par *Microbacterium trichothecenolyticum* HKMC-112 (numéro d'accès : KCCM11395P), *Microbacterium flavescens* HKMC-104 (numéro d'accès : KCCM11387P), *Methylobacterium dankookense* HKMC-101 (numéro d'accès : KCCM11384P), *Methylobacterium phyllosphaerae* HKMC-102 (numéro d'accès : KCCM11385P), *Methylobacterium tardum* HKMC-103 (numéro d'accès : KCCM11386P), *Methylobacterium radiotolerans* HKMC-111 (numéro d'accès : KCCM11394P), *Sphingomonas dokdonensis* HKMC-105 (numéro d'accès : KCCM11388P), *Sphingomonas ginsenosidimutans* HKMC-106 (numéro d'accès : KCCM11389P), *Sphingomonas humi* HKMC-107 (numéro d'accès : KCCM11390P), *Sphingomonas melonis* HKMC-108 (numéro d'accès : KCCM11391P), *Staphylococcus hominis subsp. hominis* HKMC-109 (numéro d'accès : KCCM11392P), *Staphylococcus warneri* HKMC-110 (numéro d'accès : KCCM11393P) ou une de leurs cultures ;
(b) la mise en contact d'un échantillon à analyser avec le microorganisme ;
(c) la mesure d'une inhibition de la croissance du microorganisme ou d'une diminution de la production d'odeur déplaisante ; et
(d) la détermination que l'échantillon a une activité anti-microbienne contre le microorganisme si la croissance du microorganisme est inhibée ou que la production d'odeur déplaisante est diminuée.

2. Procédé de criblage selon la revendication 1, dans lequel le système de conditionnement d'air est un conditionneur d'air.

3. Procédé de criblage selon la revendication 1 ou 2, dans lequel le microorganisme provoque une odeur déplaisante en formant un biofilm sur un coeur d'évaporateur dans le système de conditionnement d'air.

4. Procédé de criblage selon la revendication 3, dans lequel le coeur d'évaporateur est constitué d'aluminium, d'un alliage d'aluminium, de cuivre ou d'un alliage de cuivre.

5. Microorganisme provoquant une odeur déplaisante utilisé dans un système de conditionnement d'air, le microorganisme étant choisi dans le groupe constitué par *Microbacterium flavescens* HKMC-104 (numéro d'accès : KCCM11387P), *Methylobacterium dankookense* HKMC-101 (numéro d'accès : KCCM11384P), *Methylobacterium phyllosphaerae* HKMC-102 (numéro d'accès : KCCM11385P), *Methylobacterium tardum* HKMC-103 (numéro d'accès : KCCM11386P), *Methylobacterium radiotolerans* HKMC-111 (numéro d'accès : KCCM11394P), *Sphingomonas dokdonensis* HKMC-105 (numéro d'accès : KCCM11388P), *Sphingomonas ginsenosidimutans* HKMC-106 (numéro d'accès : KCCM11389P), *Sphingomonas humi* HKMC-107 (numéro d'accès : KCCM11390P), *Sphingomonas melonis* HKMC-108 (numéro d'accès : KCCM11391P), *Staphylococcus hominis subsp. hominis* HKMC-109 (numéro d'accès : KCCM11392P) et *Staphylococcus warneri* HKMC-110 (numéro d'accès : KCCM11393P).

6. Procédé d'élimination d'une odeur déplaisante dans un système de conditionnement d'air, qui comprend la séparation ou la mort d'un ou de plusieurs microorganismes provoquant une odeur déplaisante dans un système de conditionnement d'air, qui est choisi dans le groupe constitué par *Microbacterium flavescens* HKMC-104 (numéro d'accès : KCCM11387P), *Methylobacterium dankookense* HKMC-101 (numéro d'accès : KCCM11384P), *Methylobacterium phyllosphaerae* HKMC-102 (numéro d'accès : KCCM11385P), *Methylobacterium tardum* HKMC-103 (numéro d'accès : KCCM11386P), *Methylobacterium radiotolerans* HKMC-111 (numéro d'accès : KCCM11394P), *Sphingomonas dokdonensis* HKMC-105 (numéro d'accès : KCCM11388P), *Sphingomonas ginsenosidimutans* HKMC-106 (numéro d'accès : KCCM11389P), *Sphingomonas humi* HKMC-107 (numéro d'accès : KCCM11390P), *Sphingomonas melonis* HKMC-108 (numéro d'accès : KCCM11391P), *Staphylococcus hominis subsp. hominis* HKMC-109 (numéro d'accès : KCCM11392P) et *Staphylococcus warneri* HKMC-110 (numéro d'accès : KCCM11393P).

7. Procédé d'élimination d'une odeur déplaisante dans un système de conditionnement d'air, qui comprend l'inhibition d'une croissance d'un ou de plusieurs microorganismes provoquant une odeur déplaisante dans un système de conditionnement d'air, qui est choisi dans le groupe constitué par *Microbacterium flavescens* HKMC-104 (numéro d'accès : KCCM11387P), *Methylobacterium dankookense* HKMC-101 (numéro d'accès : KCCM11384P), *Methylobacterium phyllosphaerae* HKMC-102 (numéro d'accès : KCCM11385P), *Methylobacterium tardum* HKMC-103 (numéro d'accès : KCCM11386P), *Methylobacterium radiotolerans* HKMC-111 (numéro d'accès : KCCM11394P), *Sphingomonas dokdonensis* HKMC-105 (numéro d'accès : KCCM11388P), *Sphingomonas ginsenosidimutans* HKMC-106 (numéro d'accès : KCCM11389P), *Sphingomonas humi* HKMC-107 (numéro d'accès : KCCM11390P), *Sphingomonas melonis* HKMC-108 (numéro d'accès : KCCM11391P), *Staphylococcus hominis subsp. hominis* HKMC-109 (numéro d'accès : KCCM11392P) et *Staphylococcus warneri* HKMC-110 (numéro d'accès : KCCM11393P).
